# EUROPEAN PATENT SPECIFICATION

(11) **EP 1 502 961 B1**
(45) Date of publication and mention of the grant of the patent: **08.09.2010**
(21) Application number: 04017701.6
(22) Date of filing: 27.07.2004
(51) Int. Cl.: C12Q 1/68

(54) **New detection format for hot start real time polymerase chain reaction**
Neues Format zur Detektion von Heissstart- und Echtzeit-Polymerasekettenreaktion
Nouveau format de détection pour les réactions de polymerisation d'acide nucléiques en chaine avec détection en temps réel et démarrage à chaud

(30) Priority: 01.08.2003 EP 03016669
(43) Date of publication of application: 02.02.2005
(73) Proprietor: Roche Diagnostics GmbH, 68305 Mannheim (DE); F.HOFFMANN-LA ROCHE AG, 4070 Basel (CH)
(72) Inventor: Heindl, Dieter, Dr., 82396 Paehl (DE); Ankenbauer, Waltraud, Dr., 82377 Penzberg (DE); Laue, Frank, 82396 Paehl-Fischen (DE)

(56) References cited:
- EP-A- 0 744 470
- EP-A- 1 277 841
- US-A- 5 210 015
- US-A- 5 538 848
- WITTWER C T ET AL: "CONTINUOUS FLUORESCENCE MONITORING OF RAPID CYCLE DNA AMPLIFICATION" BIOTECHNIQUES, EATON PUBLISHING, NATICK, US, vol. 22, no. 1, 1997, pages 130-131,134-138, XP000683698 ISSN: 0736-6205

## Description

### Technical field

The present invention relates to the field of real time PCR. More particular, the present invention provides a new method for real time PCR, characterized amplification of a target DNA is monitored by means of hybridization with an appropriately labeled fluorescent hybridization probe in combination with a specific chemistry providing a hot start PCR effect.

### Prior art background

Amplification of DNA by polymerase chain reaction (PCR) is a technique fundamental to molecular biology. Nucleic acid analysis by PCR requires sample preparation, amplification, and product analysis. Although these steps are usually performed sequentially, amplification and analysis can occur simultaneously. DNA dyes or fluorescent probes can be added to the PCR mixture before amplification and used to analyze PCR products during amplification. Sample analysis occurs concurrently with amplification in the same tube within the same instrument. This combined approach decreases sample handling, saves time, and greatly reduces the risk of product contamination for subsequent reactions, as there is no need to remove the samples from their closed containers for further analysis. The concept of combining amplification with product analysis has become known as "real time" PCR. See, for example, U.S. Patent No. 6,174,670.

### Real Time PCR detection formats

In kinetic real time PCR, the formation of PCR products is monitored in each cycle of the PCR. The amplification is usually measured in thermocyclers which have additional devices for measuring fluorescence signals during the amplification reaction.

### a) DNA binding dye formate

Since the amount of double stranded amplification product usually exceeds the amount of nucleic acid originally present in the sample to be analyzed, double-stranded DNA specific dyes may be used, which upon excitation with an appropriate wavelength show enhanced fluorescence only if they are bound to double-stranded DNA. Preferably, only those dyes may be used which like SybrGreenI I, for example, do not affect the efficiency of the PCR reaction.

All other fomates known in the art require the design of a fluorescent labeled Hybridization Probe which only emits fluorescence upon binding to its target nucleic acid.

### b) Molecular Beacons

These hybridization probes are also labeled with a first component and with a quencher, the labels preferably being located at both ends of the probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

### c) FRET hybridization probes

The FRET Hybridization Probe test format is especially useful for all kinds of homogenous hybridization assays (Matthews, J.A., and Kricka, L.J., Analytical Biochemistry 169 (1988) 1-25). It is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected. Alternatively to monitoring the increase in fluorescence of the FRET acceptor component, it is also possible to monitor fluorescence decrease of the FRET donor component as a quantitative measurement of hybridization event.

In particular, the FRET Hybridization Probe format may be used in real time PCR, in order to detect the amplified target DNA. Among all detection formats known in the art of real time PCR, the FRET-Hybridization Probe format has been proven to be highly sensitive, exact and reliable (WO 97/46707; WO 97/46712; WO 97/46714). As an alternative to the usage of two FRET hybridization probes, it is also possible to use a fluorescent-labeled primer and only one labeled oligonucleotide probe (Bernard, P.S., et al., Analytical Biochemistry 255 (1998) 101-107). In this regard, it may be chosen arbitrarily, whether the primer is labeled with the FRET donor or the FRET acceptor compound.

### d) Single Label Probe (SLP) Format

This detection format consists of a single oligonucleotide labeled with a single fluorescent dye at either the 5'- or 3'-end (WO 02/14555). Two different designs can be used for oligo labeling: G-Quenching Probes and Nitroindole-Dequenching probes. In the G-Quenching embodiment, the fluorescent dye is attached to a C at oligo 5'- or 3'-end. Fluorescence decreases significantly when the probe is hybridized to the target, in case two G's are located on the target strand opposite to C and in position 1 aside of complementary oligonucleotide probe. In the Nitroindole Dequenching embodiment, the fluorescent dye is attached to Nitroindole at the 5'- or 3'-end of the oligonucleotide. Nitroindole somehow decreases the fluorescent signaling of the free probe. Fluorescence increases when the probe is hybridized to the target DNA due to a dequenching effect.

### e) TaqMan probe

A single-stranded Hybridization Probe is labeled with two components. When the first component is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the Taq Polymerase during the subsequent elongation phase. As a result the excited fluorescent component and the quencher are spatially separated from one another and thus a fluorescence emission of the first component can be measured. TaqMan probe assays are disclosed in detail in US 5,210,015, US 5,538,848, and US 5,487,972. TaqMan hybridization probes and reagent mixtures are disclosed in US 5,804,375.

### f) Releasing formats

Moreover, two other formats restricted to allele specific detection have been disclosed recently which are based on the principle of specific detection of a release of a labeled 3' terminal nucleotide due to a match or mismatch situation regarding its binding to the target nucleic acid. US 6,391,551 discloses a method, characterized in that the 3' terminal nucleotide of a hybridization probe is released by a depolymerizing enzyme in case a perfect match between target sequence and probe has occurred. Similarily, EP 0 930 370 suggest to use a primer labeled with a reporter and a Qunecher moiety, characterized in that a 3'-5' proofreading activity removes one moiety in case no perfect match between primer and amplification target has occurred.

### PCR Enzymology

*In vitro* nucleic acid synthesis is routinely performed with DNA polymerases with or without additional polypeptides. DNA polymerases are a family of enzymes involved in DNA replication and repair. Extensive research has been conducted on the isolation of DNA polymerases from mesophilic microorganisms such as E.coli. See, for example, Bessman,..., et al., J. Biol. Chem. 223 (1957) 171-177, and Buttin, G., and Kornberg, A., J. Biol. Chem. 241 (1966) 5419-5427.

Research has also been conducted on the isolation and purification of DNA polymerases from thermophiles, such as Thermus aquaticus. Chien, A., et al., J. Bacteriol. 127 (1976) 1550-1557 discloses the isolation and purification of a DNA polymerase with a temperature optimum of 80°C from Thermus aquaticus YT1 strain. United States Patent No. 4,889,818 discloses a purified thermostable DNA polymerase from T. aquaticus, Taq polymerase, having a molecular weight of about 86,000 to 90,000 daltons. In addition, European Patent Application 0 258 017 discloses Taq polymerase as the preferred enzyme for use in the PCR process.

Research has indicated that while Taq DNA polymerase has a 5'-3' polymerase-dependent exonuclease function, Taq DNA polymerase does not possess a 3'-5' exonuclease function (Lawyer, F.C., et al., J. Biol. Chem. 264 (1989) 6427-6437; Bernad, A., et al., Cell 59 (1989) 219-228). The 3'-5' exonuclease activity of DNA polymerases is commonly referred to as "proofreading activity". The 3'-5' exonuclease activity removes bases which are mismatched at the 3' end of a primer-template duplex. The presence of 3'-5' exonuclease activity may be advantageousas it leads to an increase in fidelity of replication of nucleic acid strands and to the elongation of prematurely terminated products . As Taq DNA polymerase is not able to remove mismatched primer ends it is prone to base incorporation errors, making its use in certain applications undesirable. For example, attempting to clone an amplified gene is problematic since any one copy of the gene may contain an error due to a random misincorporation event. Depending on the cycle in which that error occurs (e.g., in an early replication cycle), the entire DNA amplified could contain the erroneously incorporated base, thus, giving rise to a mutated gene product.

There are several thermostable DNA polymerases known in the art which exhibit 3'- 5'exonuclease activity, like B-type polymerases from thermophilic Archaebacteria which are used for high fidelity DNA amplification. Thermostable polymerases exhibiting 3'- 5'exonuclease activity may be isolated or cloned from Pyrococcus (Purified thermostable Pyrococcus furiosus DNA polymerase, Mathur E., Stratagene, WO 92/09689, US 5,545,552; Purified thermostable DNA polymerase from Pyrococcus species, Comb D. G. et al., New England Biolabs, Inc., EP 0 547 359; Organization and nucleotide sequence of the DNA polymerase gene from the archaeon Pyrococcus furiosus, Uemori, T., et al., Nucleic Acids Res. 21 (1993) 259-265), from Pyrodictium spec. (Thermostable nucleic acid polymerase, Gelfand D. H., F. Hoffmann-La Roche AG, EP 0 624 641; Purified thermostable nucleic acid polymerase and DNA coding sequences from Pyrodictium species, Gelfand D. H., Hoffmann-La Roche Inc., US 5,491,086), from Thermococcus (e.g. Thermostable DNA polymerase from Thermococcus spec. TY, Niehaus F., et al. WO 97/35988; Purified Thermocccus barossii DNA polymerase, Luhm R. A., Pharmacia Biotech, Inc., WO 96/22389; DNA polymerase from Thermococcus barossii with intermediate exonuclease activity and better long term stability at high temperature, useful for DNA sequencing, PCR etc., Dhennezel O. B., Pharmacia Biotech Inc., WO 96/22389; A purified thermostable DNA polymerase from Thermococcus litoralis for use in DNA manipulations, Comb D. G., New England Biolabs, Inc., US 5,322,785, EP 0 455 430; Recombinant thermostable DNA polymerase from Archaebacteria, Comb D. G., New England Biolabs, Inc., US 5,352,778, EP 0 547 920, EP 0 701 000; New isolated thermostable DNA polymerase obtained from Thermococcus gorgonarius, Angerer B. et al. Boehringer Mannheim GmbH, WO 98/14590.

One possibility to set up a PCR reaction with high processivity and additional proofreading activity are mixtures of Taq Polymerases and fairly trhermostable template dependent exonucleases. In this context, EP-A-1088891 discloses a thermostable enzyme obtainable from Archaeoglobus fulgidus, which catalyzes the degradation of mismatched ends of primers or polynucleotides in the 3' to 5' direction in double stranded DNA. The gene encoding the thermostable exonuclease III obtainable from Archaeoglobus fulgidus (Afu) was cloned, expressed in E.coli and isolated. The enzyme is active under the incubation and temperature conditions used in PCR reactions. The enzyme supports DNA polymerases like Taq in performing DNA synthesis at low error rates and synthesis of products of more than 3 kb on genomic DNA - the upper range of products synthesized by Taq polymerase - in good yields with or without dUTP present in the reaction mixture. Preferably, 50-500 ng of the exonuclease III obtainable from Afu were used per 2,5 U of Taq polymerase in order to have an optimal PCR performance. More preferably is the use of 67 ng to 380 ng of the exonuclease III obtainable from Afu per 2,5 U of the Taq polymerase in the PCR reaction.

### Hot start PCR

Another major problem with nucleic acid amplification and more especially with PCR is the generation of unspecific amplification products. In many cases, this is due to an unspecific oligonucleotide priming and subsequent primer extension event prior to the actual thermocycling procedure itself, since thermostable DNA polymerases are also moderately active at ambient temperature. For example, amplification products due to eventually by chance occuring primer dimerisation and subsequent extension are observed frequently. In order to overcome this problem, it is well known in the art to perform a so called "hot start" PCR, wherein one component essential for the amplification reaction is either separated from the reaction mixture or kept in an inactive state until the temperature of the reaction mixture is being raised for the first time. Since the polymerase cannot function under these conditions, there is no primer elongation during the period when the primers can bind non specifically. In order to achieve this effect, several methods have been applied:
a) Physical separation of the DNA polymeraseThe physical separation can be obtained for example by a barrier of solid wax, which separates the compartment containing the DNA polymerase from the compartment containing the bulk of the other reagents. During the first heating step the wax is then melting automatically and the fluid compartments are mixed (Chou, Q., et al., Nucleic Acids Res. 20 (1992) 1717-1723). Alternatively, the DNA polymerase is affinity immobilized on a solid support prior to the amplification reaction and only released into the reaction mixture by a heat mediated release (Nilsson, J., et al., Biotechniques 22 (1997) 744-751). Both methods, however are time consuming and unconvenient to perform.
b) Chemical modification of DNA polymerase
   For this type of hot start PCR, the DNA polymerase is reversibly inactivated as a result of a chemical modification. More precisely, heat labile blocking groups are introduced into the Taq DNA polymerase which render the enzyme inactive at room temperature. These blocking groups are removed at high temperature during a pre-PCR step such that the enzyme is becoming activated. Such a heat labile modification, for example can be obtained by coupling Citraconic Anhydride or Aconitric Anhydride to the Lysine residues of the enzyme (US 5,677,152). Enzymes carrying such modifications are meanwhile commercially availabile as Amplitaq Gold (Moretti, T., et al., Biotechniques 25 (1998) 716-722) or FastStart DNA polymerase (Roche Molecular Biochemicals). However, the introduction of blocking groups is a chemical reaction which arbitrarily occurs on all sterically available Lysine residues of the enzyme. Therefore, the reproducibility and quality of chemically modified enzyme preparations may vary and can hardly be controlled.
c) DNA polymerase inhibition by nucleic acid additives
   Extension of non-specifically annealed primers has been shown to be inhibited by the addition of short doublestranded DNA fragments (Kainz, P., et al., Biotechniques 28 (2000) 278-282). In this case, primer extension is inhibited at temperatures below the melting point of the short double stranded DNA fragment, but independent from the sequence of the competitor DNA itself. However, it is not known, to which extent the excess of competitor DNA influences the yield of the nucleic acid amplification reaction. Alternatively, oligonucleotide Aptamers with a specific sequence resulting in a defined secondary structure may be used. Such Aptamers have been selected using the SELEX Technology for a very high affinity to the DNA polymerase (US 5,693,502), (Lin, Y., and Jayasena, S.D., J. Mol. Biol. 271 (1997) 100-111). The presence of such Aptamers within the amplification mixture prior to the actual thermocycling process itself again results in a high affinity binding to the DNA polymerase and consequently a heat labile inhibition of its activity. Due to the selection process, however, all so far available Aptamers can only be used in combination with one particular species of DNA polymerase.
d) Taq DNA antibodies
   An alternative approach to achieve heat labile inhibition of Taq DNA polymerase is the addition of monoclonal antibodies raised against the purified enzyme (Kellogg, D.E., et al., Biotechniques 16 (1994) 1134-1137; Sharkey, D.J., et al., Biotechnology (N Y) 12 (1994) 506-509). Like the oligonucleotide Aptamers, the antibody binds to Taq DNA polymerase with high affinity at ambient temperatures in an inhibitory manner. The complex is resolved in a preheating step prior to the thermocycling process itself. This leads to a substantial time consuming prolongation of the amplification as a whole, especially if protocols for rapid thermocycling are applied (WO 97/46706). US 5,985,619 discloses a specific embodiment for performing PCR using a hot start antibody, wherein besides Taq polymerase, e. g. Exonuclease III from E. coli is added as a supplement to the amplification mixture in order to digest unspecific primer dimer intermediates. As disclosed above, Exonucleasae III recognizes doublestranded DNA as a substrate, like, for example, target/primer- or target/primer extension product hybrids. Digestion is taking place by means of cleavage of the phosphodiester bond at the 5' end of the 3' terminal deoxynucleotide residue. Since this type of exonuclease is active at ambient temperatures, all unspecifically annealed primers and primer extension products therefore are digested. This results in some embodiments in an even enhanced specifity of the amplification reaction. Yet, digestion of the unspecific primers dependent on the duration of the preincubation time may lead to a substantial and uncontrolled decrease in primer concentration, which in turn may affect the amplification reaction itself.
e) Usage of Exonucleases
   Another alternative for increasing amplification efficiency is the use of phosphorothioate oligonucleotide primers in combination with an exonuclease III in the PCR reaction mixes (EP 0 744 470). In this case, a 3' exonuclease, which usually accepts double stranded as well as single stranded DNA substrates, degrades duplex artefacts such as primer dimers as well as carry over amplicons, while leaving the single stranded amplification primers undegraded. In this context, it has also been suggested to use 3' bound phopshate groups which are removed upon double strand formation as a means for prevention of non template dependent primer elongation (EP 0 439 182). Similarily, the usage of primers with abasic modified 3' ends and template dependent removal by *E.coli* Endonuclease IV has been suggested (US 5,792,607). However, there exist several major draw backs of these methods:
   First, oligonucleotides containing phosphorothioate residues can not be synthesized in a stereoisomerically pure manner. Moreover, their hybridisation temperatures are different as compared to unmodified oligonucleotides of the same sequence and unspecific hybridization events are observed frequently. Second, primers containing phosphorothioate residues even at their 3' ends can still be elongated by the DNA polymerase, which is already present in the reaction mixture. In other words, the effect of the exonuclease is at least partially compensated by the presence of the polymerase itself. Third, the enzymatic acitivity of E.coli Endonuclease IV is very low in the presence of Mg++ ions (Siwek, B., et al., Nucleic Acids Res. 16 (1988) 5031-5038). Yet, dependent on the specific type of assay, an exact significant Mg++ concentration is an essentiall prerequisite for a successful PCR amplification reaction, which renders application of an endonuclease IV in a PCR sample quite ineffective. Fourth and most important, conventional nucleases like E.coli Exonuclease III or E. coli Endonuclease IV are thermolabile and therefore only active prior to the thermocycling procedure itself. As a consequence, unspecific primer binding and extension is only inhibited prior but not during the temperature cycling process.
   A further improvement of the exonuclease concept for hot start applications is disclosed in EP-A-1 277 841, which allows for an inhibition of unspecific priming and primer extension not only prior to the amplification process itself but also during the thermocycling process. In this regard, EP-A-277 841 discloses a composition for performing a nucleic acid amplification reaction comprising a thermostable DNA-Polymerase, a thermostable 3'-5' Exonuclease, and at least one primer for nucleic acid amplification with a modified 3' terminal residue which is not elongated by said thermostable DNA-Polymerase.In this context, the thermostable 3' - 5' Exonuclease is more active at temperatures between 37° C and 72° C and less active at temperatures below 37° C. The thermostable Exonuclease may either be an Exonuclease III homologue or a mutated DNA-Polymerase with no or reduced Polymerase activity.
   The concept disclosed in EP-A-1 277 841 is primarily based on the possibility to prevent primer elogation at low temperatures by introducing chemical modifications at the 3' end of at least one primer. In order to make the primer accessible at typical PCR elongation temperatures, the concept includes the use of a thermostable exonuclease which is inactive at ambient temperatures or below thus leaving the modified primer at these temperatures unaffected. Upon temperature increase, the exonuclease becomes active and capable of removing the 3' modification of the primer thus enabeling the primer to participate in the amplification reaction itself. According to the concept, the exonuclease activity is a 3'-5' exonuclease which especially recognizes such template-primer hybrids as substrates. This is the case for E.coli exonuclease III and homologues from other organisms, which recognize double stranded DNA with a 5' overhang as a preferred substrate and are especially capable of digesting the recessed 3' end of the substrate in 3'-5' direction.
   In view of the prior art discussed above, it was an object of the invention to provide an alternative economical method for real time PCR which facilitates a hot start protocol and at the same time allows for real time detection. In other words, it was an object of the present invention to develop an improved real time PCR method which does not require additional hot start additives.

### Brief description of the invention

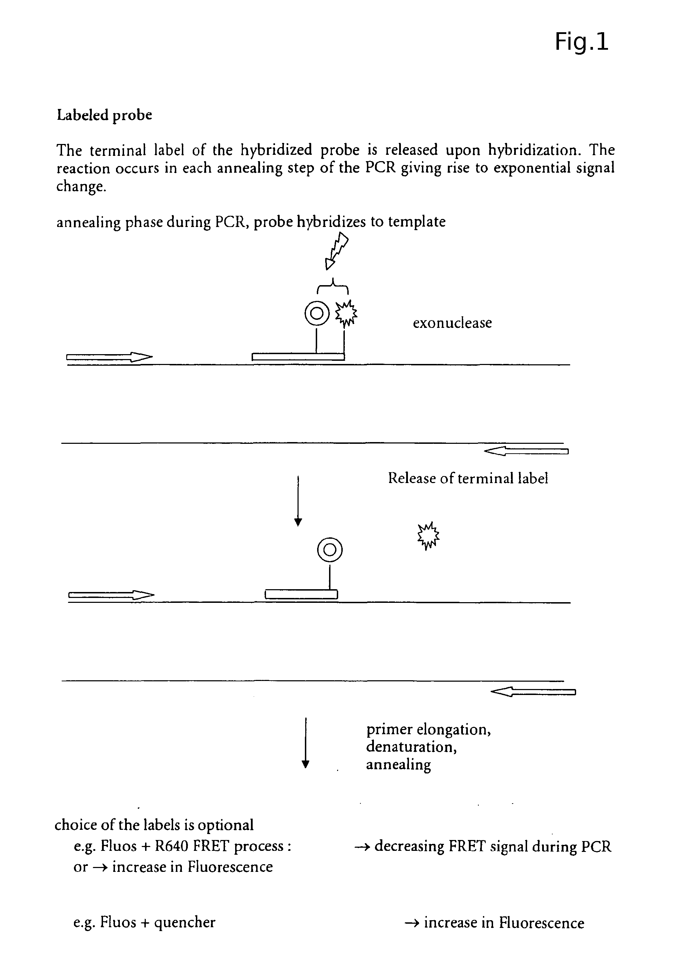

The principle underlying the present invention is schematically depicted in Fig. 1.

In a first aspect, the invention is directed to a method for amplifying and detecting a target nucleic comprising
- subjecting said target nucleic acid to a real time PCR amplification reaction in the presence of
   - a thermostable DNA Polymerase
   - a thermostable double strand dependent 3'-5' exonuclease which is not a DNA polymerase possessing 3'-5' proofreading activity
   - a pair of amplification primers as
   - Deoxynucleosidetriphosphates,
   - a hybridization probe carrying a first label and a second label,
   - said first label being capable of acting as a fluorescent reporter entity when excited with light of an appropriate wavelength,
   - said second label being capable of acting as a fluorescence quenching entity of said fluorescent reporter entity
      characterized in that one label is bound to the 3' end of said hybridization probe, and
      further characterized in that the other label is bound either internally or at the 5' end to said dehybridization probe
      further **characterized in that** said probe does not participate in the amplification process by means of priming a DNA polymerisation reaction,
   - monitoring fluorescence of said fluorerescent reporter entity at least after a plurality of amplification cycles.
   wherein a fluorescent signal is created by means of removing a 3' fluorescent entity from said hybridization probe during each cycle of amplification by said thermostable exonuclease.

In one major embodiment, the reporting entity is at the 3' end of said detecting oligonucleotide. In an alternative major embodiment, the quencher entity is at the 3' end of said detecting oligonucleotide.

Preferably, the label bound to the 3' terminal nucleotide residue of said detecting oligonucleotide is linked to said oligonucleotide via a phosphate group.

Also preferably, the second label is linked to a base of one residue of said hybridization probe. Alternatively, the second label may be linked to an abasic element of said dehybridization probe.

The double strand dependent 3'-5' exonuclease is preferably selected from a group of enzymes, siaid group consisiting of exonuclease III, endonuclease IV, DNA polymerases exhibiting 3'-5'exonuclease activity or other enzymes with 3'-5' exonuclease or proofreading activity from eucarya, prokarya, archaea, bacteriophages or viruses.

### Detailed description of the invention

As already outlined above, the present invention is directed to a method of perfroming real time PCR. It relates to a method for homogenous detection and analysis of nucleic acid sequences by means of using labeled oligonucleotides whose fluorescence changes in response to probe-target hybridization. This invention also relates to degradation of 3'ends of oligonucleotides hybridized to a DNA template, and a method for quantification of specific sequences in real-time nucleic acid amplification. Predominantly the invention is characterized in that a hybridization probe carries a fluorescent quenching entity and a fluorescent reporting entity. Similar to the TaqMan detection format, a fluorescent signal is created by means of removing a 3' fluorescent entity from said hybridization probe during each cycle of amplification.

More precisely, the present invention is directed to a method for amplifying and detecting a target nucleic comprising
- subjecting said target nucleic acid to a real time PCR amplification reaction in the presence of
   - a thermostable DNA Polymerase
   - a thermostable double strand dependent 3'-5' exonuclease which is not a DNA polymerase possessing 3'-5' proofreading activity
   - a pair of amplification primers
   - Deoxynucleosidetriphosphates,
   - a hybridization probe carrying a first label and a second label,
   - said first label being capable of acting as a fluorescent reporter entity when excited with light of an appropriate wavelength,
   - said second label being capable of acting as a fluorescence quenching entity of said fluorescent reporter entity
      characterized in that one label is bound to the 3' end of said hybridization probe, and
      further characterized in that the other label is bound either internally or at the 5' end to said hybridization probe further **characterized in that** said probe does not participate in the amplification process by means of priming a DNA polymerization reaction
   - monitoring fluorescence of said fluorerescent reporter entity at least after a plurality of amplification cycles.
wherein a fluorescent signal is created by means of removing a 3' fluorescent entity from said hybridization probe during each cycle of amplification by said thermostable exonuclease.

Prior to the thermocycling itself, only basal or substantially no fluorescent signalling occurs due to the quenching of the fluorescent reporter entity by the fluorescence quenching entity. Upon increase in temperature, the thermostable exonuclease becomes active and starts to remove the 3' terminal label provided that the hybridization probe is bound to a target molecule. As a consequence, fluorescent signalling changes during each amplification cycle due to an increase in the concentration of the amplified target DNA.

The thermostable polymerase may be any kind of DNA dependent or RNA dependent DNA polymerase, preferably Taq Polymerase from Thermus aquaticus. In a specific embodiment, a mixture of thermostable polymerases is used, wherein one polymerase Taq polymerase is providing high processivity and a second enzyme is providing a 3'-5' proofreading activity (e.g. ROCHE Cat. No.1732641).

In the context of this invention, the term "thermostable" is defined as an enzyme which retains more than 50 %, preferably more than 80% and most preferably more than 90% of its activity after 60 minutes of incubation at 70°C.

In the context of the present invention, the term " double strand dependent thermostable 3'-5' exonuclease" is defined as a nuclease which recognizes the 3' terminal recessive end of a double stranded DNA as a substrate and is capable of cleaving directly upstream (5') of the terminal phosphate group at the 3' recessive end. It is also noted that in the context of the present invention, such exonucleases are clearly disciminated from DNA polymerases possessing an additional 3'-5' proofreading activity.

The method according to the invention is also applicable for any oligonucleotide labeled at its 3' independent from the linkage between the fluorescent compound and the oligonucleotide itself, Nevertheless it is highly preferred if the label bound to the 3' terminal nucleotide residue of said hybridization probe is linked to said oligonucleotide via a phosphate group, because such a linkage facilitates a cleavage by the respective exonuclease. In case other linkers without a phosphate group are used, cleavage most probably occurs at the phosphate bonding between the terminal and the proxyterminal nucleotide residue.

Preferably, the thermostable 3' - 5' Exonuclease is more active at temperatures between 37° C and 72° C and less active at temperatures below 37° C. In other words, the enzymatic activity of the enzyme at any temperature below 37°C is in any case lower as compared to the enzymatic activity at any temperature between 37°C and 72°C. The temperature optimum for the enzyme consequently may be in the range between 50°C and 85°C.

The thermostable exonuclease is preferably an exonuclease III homologue which may be originated from any thermostable organism. In the context of this invention, an exonuclease III homologue is defined as an enzyme which recognizes double stranded DNA with a 5' overhang as a substrate and is capable of removing nucleotide residues from the 3' recesssed end. A thermostable exonuclease III homologue from Archaeoglobus fulgidus (Afu ExoIII) has been disclosed recently (EP-A-1088891), which is especially suitable for hot start protocols according to the invention. The advantage of the use of the enzyme in comparison with other enzymes is that
- the enzyme is clearly preferably active on double stranded DNA, and
- is highly active at temperatures between 37°C and 72°C but has a low activity at temperatures between 20°C and 37°C.

Alternatively, the thermostable 3' -5' exonuclease may be a mutated DNA polymerase with no or substantially reduced polymerase activity but sufficiently high 3'-exonuclease-activity. Reduced DNA polymerase activity in this context means less than 50% of said activity of an enzyme exhibiting DNA polymerase activity. Also alternativily, Endonuclease IV may be used for a mehtod according to the invention.

A hybridization probe for real time PCR monitoring itself does not participate in the amplification process by means of priming a DNA polymerization reaction.

Almost any kind of labeling is possible and, even more important, a higher degree of specificity is achieved. The disadvantage is that for a respective assay, at least 2 amplification primers and an additional dual labeled hybidization probe are required. Thus, the complexity of such an assay is increased.

In order to obtain an appropriate hot start effect, at least one or both amplification primers may be modified with a 3' terminal phosphate group or another modification which upon temperature increease is cleaved of by the thermostable exonuclease.

Furthermore, for this embodiment it has been proven to be advantageous if the hybridization probe is being prevented from an undesired extension by the thermostable DNA Polymerase during PCR. This can be obtained in two different ways:
i) If the 3' terminal label is attached to the hybridization probe via a phosphate group, the 3'-terminal nucleotide may be a so called "polymerase stopper", i. e. a nucleotide derivative or any other structure replacing a nucleotide residue which cannot be elongated by a DNA polymerase reaction. A list of putative polymerase stoppers includes base analogs, modified internucleosidic linkages and sugar analogs. Such a modification allows still cleavage of the 3'label, but after cleavage the polymerase can not recognize the generated "modified" 3 end.
ii) In case the 3' terminal label is attached to the hybridization probe via a linker that does not contain a phosphate group, the 3' proxiterminal residue is the preferred position for a "polymerase stopper".

In addition, "polymerase stoppers" as disclosed above may also prevent the detecting oligonucleotide from being completely degraded by the activity of the thermostable double strand dependent 3'-5' exonuclease.

Upon usage of a an internal "polymerase stopper" allele specific detection with a 3' labeled, discriminating 3' terminal residue may be achieved. In this case, a dual labeled oligonucleotide according to the invention in addition contains a derivatized nucleotide residue between the internal label and the 3'labeled 3' terminal residue, said derivatized nucleotide being incapable of being elongated by the thermostable DNA polymerase. If the 3' terminal discriminating nucleotide residue is matched with the target DNA, allele independent amplification and subsequent allele specific signal generation occurs due to removal of the label by the exonulcease. When the 3' terminal discriminating nucleotide residue is NOT matched with the target DNA, allele independent amplification occurs, but exonucleolytic removal of the 3' discriminating nucleotide carrying the fluorescent label is not possible and consequently, no detection signal is being generated.

As explained above, the hybridization probe according to the invention is carrying a first label and a second label. The first label is capable of acting as a fluorescent reporter entity when excited with light of an appropriate wavelength, and the said second label being capable of acting as a fluorescence quenching entity of said fluorescent reporter entity. In principle, it can be chosen abritrarily which of either the fluorescent reporter entity or the fluorescence quenching entity is bound to the 3' end of the detecting oligonucleotide and which of either of these entities is bound either internally or at the 5' end of said hybridization probe. A person skilled in the art will make his choice in this regard according to the availabiliy of fluorescent labeling reagents for any of the indicated alternatives.

Synthesis of 3' terminal modified oligonucleotides can be done by any method known in the art. Fluorescent dyes can be introduced by using a special type of commercially available controlled pore glass particles as a starter matrix for oligonucleotide synthesis. Fluorescein, for example, is disclosed in EP-A-1 186 613. In principle, the second label can be introduced at the 5' end of the hybridization probe by methods known in the art. For example, appropriate Fluorophore-Phosphoramidites may be coupled to the oligonucleotide at the end of a conventional oligonucleotide synthesis.

Preferably, however, the second label is attached internally to the oligonucleotide to provide for a close spacial vicinity between the fluorescent reporter entity and the fluorescence quenching entity as long as the 3' terminal label hasn't been removed.

In a first embodiment, said second internal label is linked to a base of one residue of said detecting oligonucleotide. An internal label can be attached at the 5 position of an internal dU (Glenn Research, Fluorescein dT10-1056-xx). Alternatively, base labeling may be done according to European application No. 03007844.8 (filed 5.4.03).

In a second embodiment, which is mutually exclusive to said first embodiment, said second label is linked to an appropriate abasic element (linker) of said hybridization probe. In this case, the abasic element is desgned in such a way that the neighbouring nucleotide residues can base pair to two complementary nucleotide residues in the target nucleic acid, which by themselves are separated from each other by 1 additional nucleotide residue. Examples are disclosed in WO 97/43451.

In a third emodiment which is also mutually exclusive to said first and said second embodiment, the label is attached to the backbone of the nucleotide chain for example by means of an appropriate Phosphthioate.

In general, any kind of Fluorescence Resonance Energy Transfer (FRET) system known in the art, which consists of a couple of a FRET donor and a FRET acceptor, can be used for providing an appropriate first label and an appropriate and an appropriate second label according to the invention. Similar if not identical to the TaqMan detection format, it is always the FRET donor compound which is excited with light of an appropriate wavelength and detected in an appropriate detection channel. For the purpose of this invention, the FRET donor is termed "fluorescent reporter entity." Consequently, the FRET acceptor compound for the purpose of this invention is called "fluorescence quenching entity". Summarizing, fluorescent reporter entities and fluorescence quenching entities which may be used for the present invention are well known by persons skilled in the art. All standard TaqMan reporter dyes such as FAM (detected at 530 nm) and HEX or VIC (detected at 560 nm) can be used. Two other specific examples are the combination Fluorescein (Reporter) and LC-Red640 (Roche Applied Science)(Quencher) or Fluorescein (Reporter) and Dabcyl (Molecular Probes) (Quencher). In a further specific embodiment, Black hole Quenchers (Biosearch Technologies) or even Nitroindole (which is known to be a quenching compound) may be used in combination with a variety of different reporter entities.

### Description of the Figures

**Figure 1**
   Schematic drawing of a real time hot start PCR reaction method according to the invention, characterized in that the detecting oligonucleotide is a hybridization probe.
**Figure 2**
   Real time monitoring of a decrease in LCRed 640 FRET signal as disclosed in example1. The primer carried an internal R640 label and a 3' terminal Fluorescein label. The terminal label was removed by exonuclease III after hybridization of the primer.
**Figure 3**
   Real time monitoring of an increase in fluorescence of Fluorescein as disclosed in example 1. The primer carries an internal R640 label and a 3' terminal Fluorescein label. The terminal label is released by exonuclease III upon hybridization of the primer leading to a change in flourescent signal.
**Figure 4**
   Gel stain of amplification products obtained in example 1.
   1. PCR reaction in the absence of exonuclease III
   2. in the presence of 33 ng
   3. in the presence of 20 ng
   4. in the presence of 12.5 ng
   5. in the presence of 5 ng of exonuclease III
   6. Molecular weight Marker V from Roche Applied Science, Cat. No. 821705
**Figure 5**
   Real Time PCR reaction as disclosed in example 2.
   "Primer 300.1 + 500rev + ProbeHPQ15: detection of product formation with a probe carrying an internal Fluorescein and a quencher molecule at the 3'end.
   "Primer 300.1 + ProbeHPQ15": detection of product formation with the help of a primer carrying an internal Fluorescein and a quencher molecule at the 3'end.

### Examples

### Example 1

The foling experiment is not an example of the invention.

For this Real time PCR experiment, using the FRET process, a dual labeled primer was designed which carried an internal LC-Red640 label (Roche Applied Science Cat. No. 2 015 161) and a 3' terminal Fluorescein label (Roche Applied Science Cat. No. 3 138 178). The terminal label was removed by exonuclease III after hybridization of the primer which resulted in a decrease in LCRed640 signalling and at the same time in an increase in Fluorescein fluorescence.

Primers were as follows:
Seq. Id No.1:
   "ßActin5.2fd": GGATTCCTATGTGGGCGACG
Seq. Id.No. 2:
   "ßActinHP25": CCTGGGTCATCTTCT**(Red 640)CGCGG*U*TpFluos-3'
   T**(Red640)=T-LCRed640 (Amino-modified T-Phosphoramidate was introduced during oligonucleotide synthesis. Subsequently, the reactive amino group was reacted with LC-Red640 NHS ester)
   U*:= 2'-O-Methyl-U
   G*= 2'-O-Methyl-G
   p= phosphate
   Fluos= Fluorescein

Synthesis and labeling of the oligonucleotides were performed according to standard protocols known in the art.

PCR reactions were setup with 2 µl LightCycler FastStart Reaction Mix Hybridization Probes (Roche Applied Science Cat.No. 3003248), 30 ng of human genomic DNA (Roche Applied Science Cat.No. 1691112), 3 mM MgCl₂, 500 nM Primer seqences No. β*Actin5.2fd*, 400 nM Primer Seq.No. β*ActinHP25* and 2.5 units of Taq polymerase without A. fulgidus exonuclease III and with addition of decreasing amounts of A. fulgidus exonuclease III, 33 ng, 20 ng, 12.5 ng and 5 ng per reaction. The final reaction volume of 20 µl was adjusted with destilled water. The PCR reactions were performed on a LightCycler (Roche Applied Science Cat.No. 2011468) programed according to the instructions of the manufacturer's user manual. PCR conditions were as follows:

| 1 cycle | |
|---|---|
| 60 sec | 95°C |

| 45 cycles | |
|---|---|
| 0 sec | 95°C |
| 10 sec | 60°C |
| 15 sec | 72 °C |

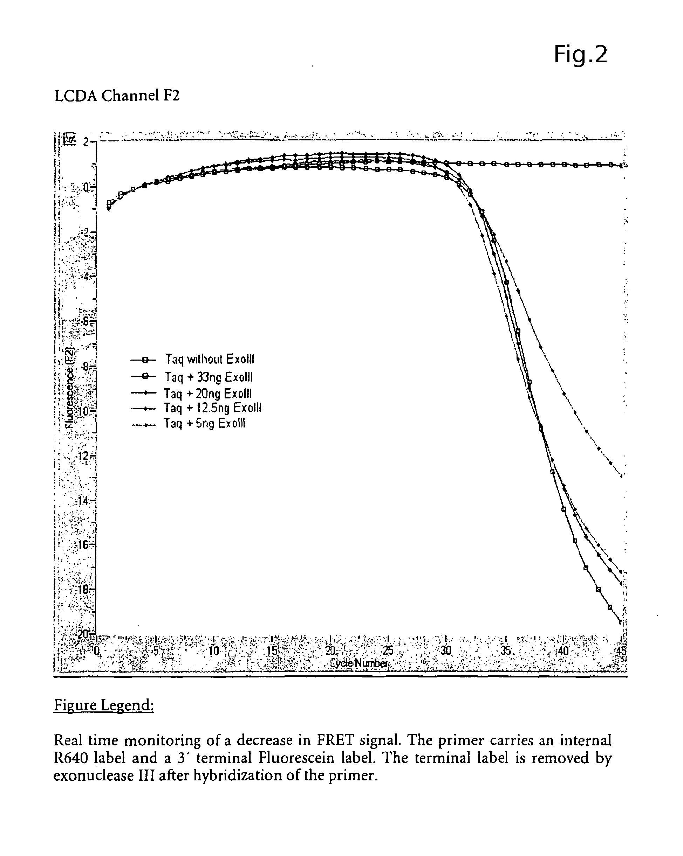

Results are shown in Figures. 2, 3, and 4. As can be seen in Fig. 2, the LCRed640 signal monitored in Channel F2 decreased with increasing cycle number. The effect was dose dependent with respect to the amount of A. fulgidus exonuclease III in the reaction mixture. In the absence of exonuclease III no decrease in LC-red640 fluorescence was observed.

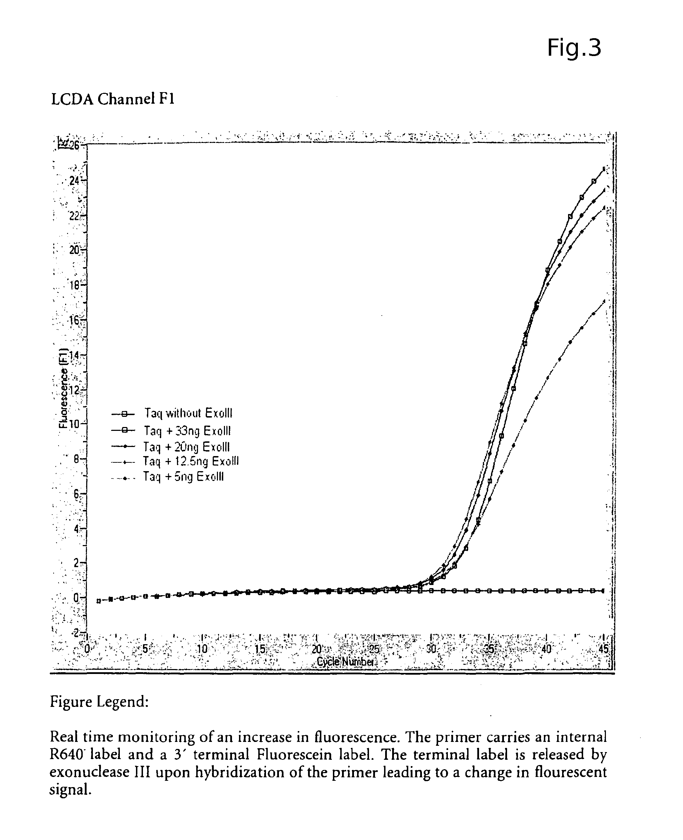

The Fluorescence emission of Fluorescein was monitored in Channel F1. As can be seen in Fig. 3, the signal increased with increasing cycle number. The effect was dose dependent witrh respect to the amount of A. fulgidus exonuclease III in the reaction mixture. In the absence of exonuclease III no increase in Fluorescein signaling was observed.

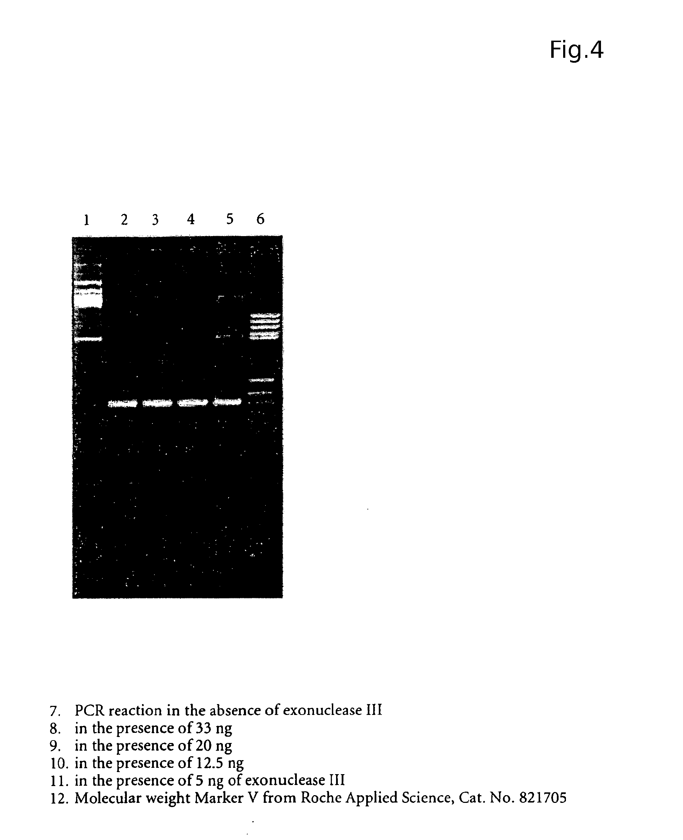

After completion of the LightCycler analysis the PCR products were analyzed on a 3% ROCHE Agarose MS gel stained with Ethidiumbromide. The result are shown in Fig. 4. Only in the presence of A. fulgidus exonuclease III the expected PCR product of 214 bp could be detected (lanes 2-5). **Example**

In another real time PCR experiment, the reaction was monitored with an oligonucleotide probe carrying an internal Fluorescein and Dabcyl as a 3' terminal quencher compound. After hybridization of the oligonucleotide the terminal quencher was removed by exonuclease III which resulted in a dequenching of the fluorescein signal. The reporter groups awere either located on an oligonucleotide used either as a probe (Reaction 1, "Primer 300.1 + 500rev + ProbeHPQ15") or, alternatively as a primer (Reaction 2, "Primer 300.1 + ProbeHPQ15").

Primer and probe sequences were as follows:
SEQ ID NO: 3
   "Primer βAct300.1" CACCCCGTGCTGCTGACCGAp
   p= phosphate
   SEQ. ID. No: 4
   "β-Act 500 rev" AGGGAGGCGGCCACCAGAAGp
   p=phosphate
SEQ. ID. No: 5
   "βActinHPQ15" CCTGGGTCATCTTCT**(Fluos)CGCGGTTpZ
   p=phosphate
   Z=Dabcyl
   T**(Fluos)= T-Fluorescein, incorporated during oligonucleotide synthesis as T-Fluorescein-Phosphoramidite.

PCR reactions were setup with 2 µl LightCycler FastStart Reaction Mix Hybridization Probes (Roche Applied Science Cat.No. 3003248), 30 ng of human genomic DNA (Roche Applied Science Cat.No. 1691112), 3 mM MgCl₂, 2.5 units of Taq polymerase, 33 ng of A. fulgidus exonuclease III. Reaction No 1 contained 500 nM of *Primer Seq 300.1,* 500 nM of Primer *500 rev* and 500nM of Probe *Sequ. No HPQ15*. Reaction No. 2 contained 500 nM of Primer *300.1* and 500 nM of *βActin HPQ15*. The final reaction volume of 20 µl was adjusted with destilled water. The PCR reactions werde performed on a LightCycler (Roche Applied Science Cat.No. 2011468) programed according to the instructions of the manufacturer's user manual. PCR conditions were as follows:

| 1 cycle | |
|---|---|
| 60 sec | 95°C |

| 45 cycles | |
|---|---|
| 0 sec | 95°C |
| 10 sec | 65°C |
| 10 sec | 72 °C |

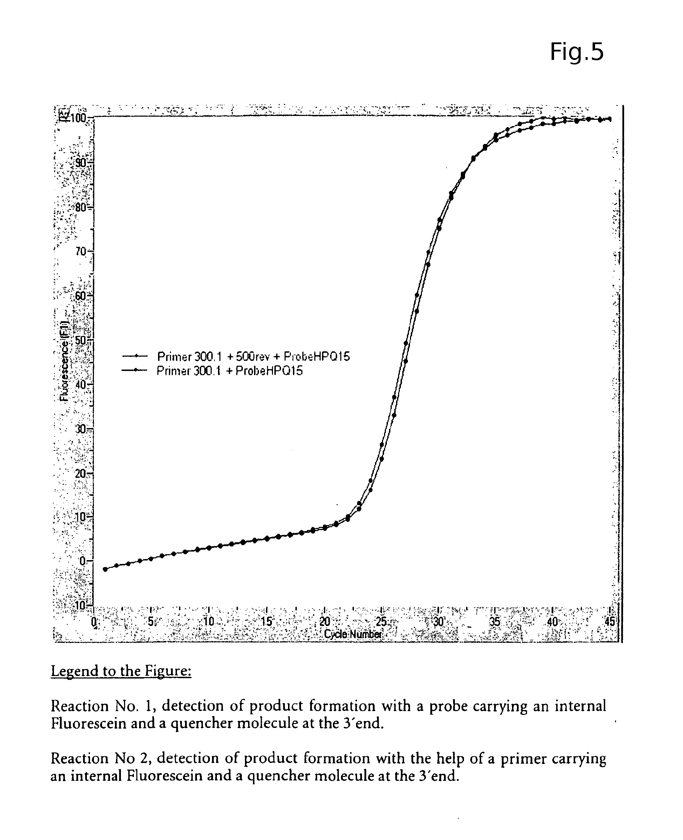

The real time PCR reactions were monitored in Channel F1 and an increase in signal was observed with increasing PCR product formation. As it is shown in Figure 5, both, use of a double labeled probe according to the invention (Reaction 1, "Primer 300.1 + 500rev + ProbeHPQ15") as well as use of a double labeled primer according to the invention (Reaction 2, "Primer 300.1 + ProbeHPQ15) resulted insuccessful amplification signaling.

### List of References

Bernad, A., et al., Cell 59 (1989) 219-228
Bernard, P.S., et al., Analytical Biochemistry 255 (1998) 101-107
Bessman, M.J., et al., J. Biol. Chem. 223 (1957) 171-177
Buttin, G., and Kornberg, A., J. Biol. Chem. 241 (1966) 5419-5427
Chien, A., et al., J. Bacteriol. 127 (1976) 1550-1557
Chou, Q., et al., Nucleic Acids Res. 20 (1992) 1717-1723
EP 0 258 017
EP 0 3 007 844.8
EP 0 439 182
EP 0 455 430
EP 0 547 359
EP 0 547 920
EP 0 624 641
EP 0 701 000
EP 0 744 470
EP 0 930 370
EP-A-1 088 891
EP-A-1 186613
EP-A-1 277 841
EP-A-277 841
Kainz, P., et al., Biotechniques 28 (2000) 278-282
Kellogg, D.E., et al., Biotechniques 16 (1994) 1134-1137
Lawyer, F.C., et al., J. Biol. Chem. 264 (1989) 6427-6437
Lin, Y., and Jayasena, S.D., J. Mol. Biol. 271 (1997) 100-111
Matthews, J.A., and Kricka, L.J., Analytical Biochemistry 169 (1988) 1-25
Moretti, T., et al., Biotechniques 25 (1998) 716-722
Nilsson, J., et al., Biotechniques 22 (1997) 744-751
Sharkey, D.J., et al., Biotechnology 12 (1994) 506-509
Siwek, B., et al., Nucleic Acids Res. 16 (1988) 5031-5038
Uemori, T., et al., Nucleic Acids Res. 21 (1993) 259-265
US 4,889,818
US 5,118,801
US 5,210,015
US 5,322,785
US 5,352,778
US 5,487,972
US 5,491,086
US 5,538,848
US 5,545,552
US 5,677,152
US 5,693,502
US 5,792,607
US 5,804,375
US 5,985,619
US 6,391,551
WO 02/14555
WO 92/09689
WO 96/22389
WO 97/35988
WO 97/43451
WO 97/46706
WO 97/46707
WO 97/46712
WO 97/46714
WO 98/14590

## Claims

1. A method for amplifying and detecting a target nucleic comprising
- subjecting said target nucleic acid to a real time PCR amplification reaction in the presence of
- a thermostable DNA Polymerase
- a thermostable double strand dependent 3'-5' exonuclease which is not a DNA polymerase possessing 3'-5' proofreading activity
- a pair of amplification primers
- Deoxynucleoside-Tri-Phosphates,
- a hybridization probe carrying a first label and a second label ,
- said first label being capable of acting as a fluorescent reporter entity when excited with light of an appropriate wavelength,
- said second label being capable of acting as a fluorescence quenching entity of said fluorescent reporter entity
**characterized in that** one label is bound to the 3' end of said hybridization probe, and
further **characterized in that** the other label is bound either internally or at the 5' end to said hybridization probe,
further **characterized in that** said probe does not participate in the amplification process by means of priming a DNA polymerization reaction - monitoring fluorescence of said fluorerescent reporter entity at least after a plurality of amplification cycles,
- wherein a fluorescent signal is created by means of
removing a 3' fluorescent entity from said hybridization probe during each cycle of amplification by said thermostable exonuclease.

2. Method according to claim 1,
wherein said reporting entity is at the 3' end of said hybridization probe.

3. Method according to claim 1-2,
wherein said label bound to the 3' terminal nucleotide residue of said hybridization probe is linked to said oligonucleotide via a phosphate group.

4. Method according to claims 1-3
wherein said double strand dependent 3'-5' exonuclease is selected from a group consisting of Exonuclease III and Endonuclease IV.

5. Method according to claims 1-4,
wherein said second label is linked to a base of one residue of said hybridization probe.

6. Method according to claims 1-5
wherein said second label is linked to an abasic element of said hybridization probe.

## Patentansprüche

1. Verfahren zum Amplifizieren und Nachweisen einer Zielnukleinsäure, bei dem man
- die Zielnukleinsäure einer Echtzeit-PCR-Amplifikationsreaktion in Gegenwart
- einer thermostabilen DNA-Polymerase,
- einer thermostabilen Doppelstrang-abhängigen 3'-5'-Exonuklease, bei der es sich nicht um eine DNA-Polymerase mit 3'-5'-Proofreading-Aktivität handelt,
- einem Amplifikations-Primer-Paar,
- Desoxynukleosidtriphosphaten,
- einer eine erste und eine zweite Markierung tragenden Hybridisierungssonde,
- wobei die erste Markierung bei Anregung mit Licht einer entsprechenden Wellenlänge als Fluoreszenzreportereinheit fungieren kann,
- wobei die zweite Markierung als Fluoreszenz-Quencher-Element der Fluoreszenzreportereinheit fungieren kann,
unterzieht, **dadurch gekennzeichnet, dass** eine Markierung an das 3'-Ende der Hybridisierungssonde gebunden ist und
ferner **dadurch gekennzeichnet, dass** die andere Markierung entweder intern oder am 5'-Ende an die Hybridisierungssonde gebunden ist,
ferner **dadurch gekennzeichnet, dass** die Sonde nicht an dem Amplifikationsvorgang mittels Priming einer DNA-Polymerisationsreaktion beteiligt ist,
- Beobachten der Fluoreszenz der Fluoreszenzreportereinheit wenigstens nach mehreren Amplifikationszyklen,
- wobei ein Fluoreszenzsignal mittels Abtrennen einer 3'-Fluoreszenzeinheit von der Hybridisierungssonde durch die thermostabile Exonuklease während eines jeden Amplifikationszyklus erzeugt wird.

2. Verfahren nach Anspruch 1,
wobei die Reportereinheit am 3'-Ende der Hybridisierungssonde liegt.

3. Verfahren nach Anspruch 1-2,
wobei die an den 3'-terminalen Nukleotidrest der Hybridisierungssonde gebundene Markierung mit dem Oligonukleotid über eine Phosphatgruppe verknüpft ist.

4. Verfahren nach Anspruch 1-3,
wobei die Doppelstrang-abhängige 3'-5'-Exonuklease aus einer aus Exonuklease III und Endonuklease IV bestehenden Gruppe ausgewählt ist.

5. Verfahren nach Anspruch 1-4,
wobei die zweite Markierung mit einer Base eines Restes der Hybridisierungssonde verknüpft ist.

6. Verfahren nach Anspruch 1-5,
wobei die zweite Markierung mit einem abasischen Element der Hybridisierungssonde verknüpft ist.

## Revendications

1. Procédé pour l'amplification et la détection d'un acide nucléique cible, comprenant :
- le fait de soumettre ledit acide nucléique cible à une réaction d'amplification par PCR en temps réel en présence
- d'une ADN polymérase thermostable
- d'une exonucléase 3'-5' dépendante de double brin thermostable qui ne représente pas d'ADN polymérase possédant une activité de correction d'épreuves 3'-5' ;
- d'une paire d'amorces d'amplification ;
- de désoxynucléoside-triphosphates ;
- d'une sonde d'hybridation portant un premier marqueur et un deuxième marqueur ;
- ledit premier marqueur étant à même de faire office d'entité rapporteur fluorescente lorsqu'il est excité avec de la lumière possédant une longueur d'onde appropriée ;
- ledit deuxième marqueur étant à même de faire office d'entité d'extinction de la fluorescence de ladite entité rapporteur fluorescente ;
**caractérisé en ce qu'**un marqueur est lié à l'extrémité 3' de ladite sonde d'hybridation ; et
**caractérisé en outre en ce que** l'autre marqueur est lié, soit à l'intérieur, soit à l'extrémité 5' de ladite sonde d'hybridation ;
**caractérisé en outre en ce que** ladite sonde ne participe pas au processus d'amplification par amorçage d'une réaction de polymérisation d'ADN ;
le fait de surveiller la fluorescence de ladite entité rapporteur fluorescente au moins après plusieurs cycles d'amplification ;
dans lequel un signal fluorescent est généré en éliminant une entité fluorescente 3' de ladite sonde d'hybridation au cours de chaque cycle d'amplification via ladite exonucléase thermostable.

2. Procédé selon la revendication 1, dans lequel ladite entité rapporteur se trouve à l'extrémité 3' de ladite sonde d'hybridation.

3. Procédé selon les revendications 1-2, dans lequel ledit marqueur lié au résidu nucléotidique de l'extrémité 3' de ladite sonde d'hybridation est lié audit oligonucléotide via un groupe phosphate.

4. Procédé selon les revendications 1-3, dans lequel ladite exonucléase 3'-5' dépendante de double brin est choisie parmi le groupe constitué par l'exonucléase III et l'endonucléase IV.

5. Procédé selon les revendications 1-4, dans lequel ledit deuxième marqueur est lié à une base d'un résidu de ladite sonde d'hybridation.

6. Procédé selon les revendications 1-5, dans lequel ledit deuxième marqueur est lié à un élément abasique de ladite sonde d'hybridation.


### SEQUENCE LISTING

<110> Roche Diagnostics GmbH
   F. Hoffmann-La Roche AG
<120> New detection format for hot start real time Polymerase Chain
   Reaction
<130> 21869 EP
<160> 5
<170> PatentIn version 3.2
<210> 1
   <211> 20
   <212> DNA
   <213> Artificial
<220>
   <223> primer
<400> 1
   ggattcctat gtgggcgacg 20
<210> 2
   <211> 22
   <212> DNA
   <213> Artificial
<220>
   <223> primer
<400> 2
   cctgggtcat cttctcgcgg tt 22
<210> 3
   <211> 20
   <212> DNA
   <213> Artificial
<220>
   <223> primer
<400> 3
   caccccgtgc tgctgaccga 20
<210> 4
   <211> 20
   <212> DNA
   <213> Artificial
<220>
   <223> primer
<400> 4
   agggaggcgg ccaccagaag 20
<210> 5
   <211> 22
   <212> DNA
   <213> Artificial
   <220>
   <223> primer
<400> 5
   cctgggtcat cttctcgcgg tt 22